(19) **Europäisches Patentamt**

**European Patent Office**

**Office européen des brevets**

(11) **EP 1 193 488 B1**

(12) **EUROPÄISCHE PATENTSCHRIFT**

(45) Veröffentlichungstag und Bekanntmachung des Hinweises auf die Patenterteilung:
**08.12.2004 Patentblatt 2004/50**

(51) Int Cl.$^7$: **G01N 21/31**, G01N 33/22, G01N 21/35

(21) Anmeldenummer: **01123027.3**

(22) Anmeldetag: **26.09.2001**

(54) **Verfahren und Vorrichtung zum Ermitteln der Gasbeschaffenheit eines Erdgases**

Method and device for determining the gaseous quality of natural gas

Procede et dispositif pour determiner la nature de gaz naturel

(84) Benannte Vertragsstaaten:
**AT BE CH CY DE DK ES FI FR GB GR IE IT LI LU MC NL PT SE TR**

(30) Priorität: **29.09.2000 DE 10048839**
**03.05.2001 DE 10121641**

(43) Veröffentlichungstag der Anmeldung:
**03.04.2002 Patentblatt 2002/14**

(73) Patentinhaber: **E.ON Ruhrgas AG**
**45138 Essen (DE)**

(72) Erfinder: **Schley, Peter Dr.**
**45133 Essen (DE)**

(74) Vertreter: **Harlacher, Mechthild et al**
**E.ON Ruhrgas AG,**
**Huttropstrasse 60**
**45138 Essen (DE)**

(56) Entgegenhaltungen:
**EP-A- 0 882 977          EP-A- 0 967 483**
**DE-A- 19 535 819          DE-A- 19 650 302**
**DE-A- 19 921 167          US-A- 4 594 510**

- **LUEPTOW R M ET AL: "ACOUSTIC SENSOR FOR DETERMENING COMBUSTION PROPERTIES OF NATURAL GAS" MEASUREMENT SCIENCE AND TECHNOLOGY, IOP PUBLISHING, BRISTOL, GB, Bd. 5, Nr. 11, 1. November 1994 (1994-11-01), Seiten 1375-1381, XP000483676 ISSN: 0957-0233**

**Beschreibung**

Die Erfindung betrifft ein Verfahren und eine Vorrichtung zum Ermitteln der Gasbeschaffenheit eines Brenngases. Unter Gasbeschaffenheit wird die Gaszusammensetzung, der Brennwert, die Wobbezahl bzw. der Wobbeindex, die Normdichte und die Methanzahl verstanden.

Der Brennwert kann der molare, der massenbezogene oder der volumenbezogene Brennwert sein. Der Brennwert von Erdgas muss zu Abrechnungszwecken bei der Übergabe vom Lieferanten auf den Kunden gemessen werden. An Übergabestationen, beispielsweise zwischen zwei Gasversorgungsunternehmen, wird der Brennwert in der Praxis mittels Kalorimetern oder Gaschromatographen ermittelt. Bei dem letztgenannten Verfahren wird die Gaszusammensetzung analysiert. Aus der Zusammensetzung des Gases kann dann mit dem Brennwert für die reinen Stoffe der Brennwert des Brenngases bestimmt werden. Mit Gaszählern, insbesondere Turbinenradzählern, wird der Volumenstrom gemessen. Der Volumenstrom muss mit Hilfe der Kompressibilitätszahl vom Betriebs- auf den Normzustand umgewertet werden. Die Kompressibilitätszahl lässt sich nach dem bekannten SGERG Verfahren (ISO12213) aus Brennwert, Normdichte und $CO_2$-Anteil berechnen. Wird der Brennwert mittels Kalorimeter bestimmt, so müssen die Normdichte und der $CO_2$-Anteil zusätzlich gemessen werden. Wird ein Gaschromatograph eingesetzt, so können Normdichte und $CO_2$-Anteil aus der Gaszusammensetzung berechnet werden. Die Energiemenge ergibt sich dann über das Produkt aus Brennwert und Normvolumenstrom.

Die Verfahren zur Ermittlung des Brennwertes mittels Kalorimetern oder Gaschromatographen liefern sehr gute Ergebnisse, sind aber technisch kompliziert und verursachen sehr hohe Investitions- und Wartungskosten. Für verschiedene industrielle Anwendungen, insbesondere für Regelungszwecke sind derartige Verfahren zu aufwendig und bezüglich der Ansprechzeit zu träge.

Zu Abrechnungszwecken im Hochdruckbereich sind auch korrelative Verfahren zur Ermittlung des Brennwertes bzw. der Energiemenge bekannt. Bei diesen korrelativen Verfahren werden mehrere physikalische oder chemische Größen gemessen und der Brennwert berechnet.

Aus der DE 197 36 528 und der DE 198 08 533 sind korrelative Verfahren bekannt, bei denen als Eingangsgrößen u. a. die Schallgeschwindigkeit und die Dielektrizitätskonstante des Brenngases gemessen werden. Aus diesen Messsignalen wird der Brennwert oder die Gaszusammensetzung berechnet.

Die Schallgeschwindigkeit kann von einem Ultraschall-Volumenstromzähler abgegriffen werden. Solche Zähler, die vorwiegend im Hochdruck eingesetzt werden, sind jedoch vergleichsweise teuer. Preiswertere Ultraschall-Volumenstromzähler wurden für den haushaltlichen Bereich entwickelt. Jedoch konnten sich diese Zähler gegenüber dem herkömmlichen Balgengaszähler auf dem Markt bisher nicht behaupten. Demnach ist die zukünftige Verfügbarkeit des Ultraschallzähler-Haushaltszähler fragwürdig. Die Dielektrizitätskonstante muss mit einem speziell zu diesem Zweck entwickelten Messgerät bestimmt werden. Folglich ist der messgerätetechnische Aufwand relativ groß.

Die Kenntnis der Gasbeschaffenheitsgrößen, insbesondere des Brennwertes oder der Wobbezahl wird auch für verschiedene industrielle Anwendungen benötigt, insbesondere zu Regelungszwecken.

Die Wobbezahl bzw. der Wobbeindex ist der Quotient des volumenbezogenen Brennwertes und der Quadratwurzel aus der relativen Dichte des Gases. Der Wobbeindex wird in der Industrie zur Regelung bzw. Konstanthaltung der Energiemengenzufuhr zu Gasverbrauchseinrichtungen benutzt. Ein einfaches korrelatives Verfahren für derartige Zwecke steht bisher nicht zur Verfügung.

Unter Normdichte wird die massenbezogene und die molare Normdichte verstanden. In der Praxis wird in der Regel die massenbezogene Normdichte verwendet, insbesondere zur Berechnung der Kompressibilitätszahl.

Die Methanzahl ist im Zusammenhang mit Gasmotoren eine wichtige Kenngröße. Die Methanzahl ist ein Maß für die Klopffestigkeit von gasförmigen Brennstoffen. Die Methanzahl gibt den prozentualen Methananteil einer Methan/ Wasserstoff-Mischung an, die in einem Prüfmotor unter definierten Randbedingungen dieselbe Klopfstärke aufweist wie das zu untersuchende Gas. Wenn z. B. ein Erdgas eine Methanzahl von 85 besitzt, so heißt dies, dass unter bestimmten motorischen Bedingungen dieses Erdgas die gleiche Klopfstärke zeigt wie ein Gemisch aus 85% Methan und 15% Wasserstoff. Bei Kenntnis der Methanzahl kann das unerwünschte Motorklopfen von erdgasbetriebenen Kolbenmotoren durch entsprechende Maßnahmen vermieden werden.

Ein Verfahren zur Bestimmung der Methanzahl ist aus der DE-A-19650302 bekannt. Das Brenngas wird einer Infrarotstrahlung ausgesetzt. Mittels eines Strahlungsdetektors wird der von der Gasmischung absorbierte Anteil der Infrarotstrahlung gemessen und hieraus die Methanzahl des Brenngases bestimmt.

Die Bestimmung der Methanzahl erfolgt über einen optischen Filter, der einen Ausschnitt des Absorptionsspektrums erfasst, in dem die Kohlenwasserstoffe in einer Gewichtung zur Absorption beitragen, die näherungsweise proportional zur Methanzahl des Erdgases ist. Das Verfahren kann relativ einfach in die Praxis umgesetzt werden, weil zum einen die Komponenten der entsprechenden Infrarotsensoren preiswert am Markt erhältlich sind und zum anderen die Infrarotdetektoren ein sehr präzises Messsignal liefern und eine gute Praxistauglichkeit besitzen.

Eine Bestimmung des Brennwertes von Erdgasen mittels Infrarotabsorption konnte mit den bisher bekannten Verfahren technisch nicht realisiert werden. Die verschiedenen Erdgase können neben Kohlenwasserstoffen wie Methan, Ethan usw. auch Stickstoff enthalten. Das Infrarotsignal reagiert - in Abhängigkeit des gefilterten Absorptionsspektrums - sehr empfindlich auf die Anteile der Kohlenwasserstoffe und auf den Kohlendioxidanteil, nicht jedoch auf den Stickstoffanteil. Dies führt zu nicht vertretbaren Messungenauigkeiten; denn der Stickstoffanteil im Erdgas unterliegt großen Schwankungen und hat einen großen Einfluss auf den Brennwert.

Aufgabe der Erfindung ist es demgemäss, ein Verfahren zur verbrennungslosen Bestimmung der Gasbeschaffenheit, insbesondere des Brennwertes, der Wobbezahl und der Methanzahl eines Brenngases zur Verfügung zu stellen, das zum einen einfach realisiert werden kann und zum anderen eine ausreichende Genauigkeit für Abrechnungs- und für Regelungszwecke bietet. Aufgabe der Erfindung ist es weiterhin, eine einfache und praktisch einsetzbare Messanordnung zu schaffen.

Bei dem erfindungsgemäßen Verfahren zur Bestimmung der Gasbeschaffenheit wird zumindest ein Teil des Brenngases einer Infrarotstrahlung ausgesetzt und für zwei unterschiedliche Wellenlängen oder spektrale Bereiche jeweils der von dem Brenngas absorbierte Anteil der Infrarotstrahlung vorzugsweise mit jeweils einem Infrarotsensor gemessen. Unter Wellenlänge wird die Zentralwellenlänge verstanden. Der spektrale Bereich wird durch die Zentralwellenlänge und die Halbwertsbreite charakterisiert. Zusätzlich wird mit einem Wärmeleitfähigkeitssensor die Wärmeleitfähigkeit gemessen. Aus den Signalen der Infrarotsensoren, das heißt den beiden Messwerten für den von dem Brenngas absorbierten Anteil der Infrarotstrahlung und dem Signal des Wärmeleitfähigkeitssensor wird die Gasbeschaffenheit rechnerisch bestimmt.

Die Wellenlängen oder spektralen Bereiche werden derart ausgewählt, dass sich die Anteile einzelner Komponenten des Brenngases in unterschiedlicher Gewichtung auf die erfassten absorbierten Anteile auswirken.

Mit einem der Infrarotsensoren wird direkt der Stoffmengenanteil des Kohlendioxids im Erdgas bestimmt. Dieser Sensor arbeitet vorzugsweise bei einer Wellenlänge von 4,3 μm.

Der zweite Infrarotsensor, der vorzugsweise bei einer Wellenlänge von 3,5 μm arbeitet, erfasst die Kohlenwasserstoffe im Erdgas. Die Wellenlänge wurde so gewählt, dass der Sensor eine möglichst große Empfindlichkeit bezüglich der Kohlenwasserstoffe aufweist.

Die Wärmeleitfähigkeit weist dagegen eine starke Empfindlichkeit gegenüber Stickstoff auf.

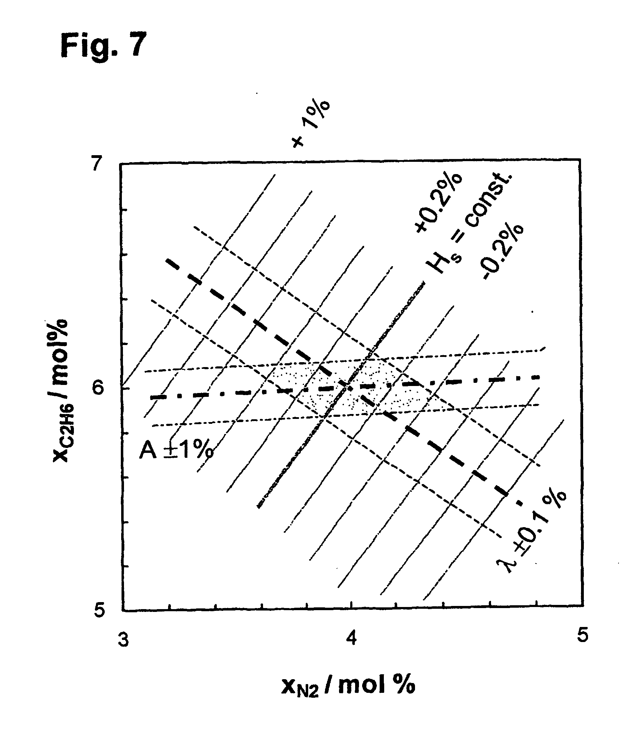

Die verschiedenen Sensoren liefern somit drei Signale, die sehr unterschiedlich auf die verschiedenen Komponenten bzw. Gruppen von Komponenten reagieren und somit nicht miteinander korrelieren. Dieser Sacherhalt, der auch durch die grafische Darstellung in Fig. 7 veranschaulicht wird, ist die Grundvoraussetzung dafür; dass das erfindungsgemäße Verfahren für eine große Vielfalt von Erdgasen eine hohe Genauigkeit liefert. Mit alleinigen Infrarotstrahlungs-Messungen, die keine bzw. nur eine sehr schwache Empfindlichkeit gegenüber Stickstoff aufweisen, kann eine solche Genauigkeit nicht erreicht werden.

Durch das beschriebene Verfahren werden die verschiedenen zuvor beschriebenen Anwendungen zur Gasbeschaffenheitsbestimmung, d. h. Energiemessung (Brennwert, Normdichte, $CO_2$-Anteil) und Prozesssteuerung (Wobbezahl / Methanzahl) gleichzeitig erschlossen. Die erreichbare Genauigkeit ist vergleichbar mit der Genauigkeit von zur Abrechnung eingesetzten Kalorimetern oder Prozessgaschromatographen. Dabei ist das beschriebene Verfahren wesentlich kostengünstiger und erfordert deutlich weniger Wartungsaufwand.

Für die Bestimmung der unterschiedlichen Messgrößen können prinzipiell verschiedene Sensortypen verwendet werden. Allerdings liefert jeder Sensortyp jeweils typspezifische Messwerte. Es wurde nun durch Versuche gefunden, dass sich aus den Sensorsignalen eine einfache Korrelation zur Gasbeschaffenheit ableiten lässt. Zum Aufbau der Korrelation werden insbesondere verschiedene empirische Zusammenhänge ausgenutzt, die durch Labormessungen an Methan sowie einer Reihe von natürlichen Erdgasen ermittelt wurden. Zum einen wurde der funktionale Zusammenhang zwischen dem Brennwert $H_{CH}$ der Kohlenwasserstoffe und dem Quotienten aus Infrarotabsorption A und Stoffmengenanteil $x_{CH}$ der Kohlenwasserstoffe aufgestellt.

Außerdem wurde erkannt, dass die Wärmeleitfähigkeit $\lambda_{CH}$ des Kohlenwasserstoffgases eine Funktion des Quotienten aus Infrarotabsorption A und des Stoffmengenanteils $x_{CH}$ der Kohlenwasserstoffe ist. In ähnlicher Weise wurde der funktionale Zusammenhang zwischen der molaren Normdichte $\rho_{mn,CH}$ und dem Quotienten aus Infrarotabsorption A und des Stoffmengenanteils $x_{CH}$ der Kohlenwasserstoffe erkannt.

Die Kennlinien müssen für einen bestimmten Sensortypen nur einmalig aufgenommen werden. Zur späteren Kalibrierung genügt eine Punktweise Überprüfung mit einem reinen Gas Wie z.B. Methan.

Die Genauigkeit des Verfahrens kann erhöht werden, wenn zusätzlich der vom Brenngas absorbierte Anteil der Infrarotstrahlung bei einer weiteren Zentralwellenlänge von 7,9 μm erfasst wird. Bei dieser Wellenlänge reagiert der Sensor besonders empfindlich auf den Methananteil im Brenngas. Weiterhin besteht die Möglichkeit, mit dieser zusätzlichen Messung ein redundantes System zu Prüfzwecken aufzubauen.

Vorzugsweise wird der Anteil der Infrarotstrahlung sowie die Wärmeleitfähigkeit unter Referenzbedingungen in einer gemeinsamen Messumgebung erfasst.

Vorteilhafterweise werden im Schritt a) oder b) die Temperatur und der Druck erfasst oder konstant gehalten.

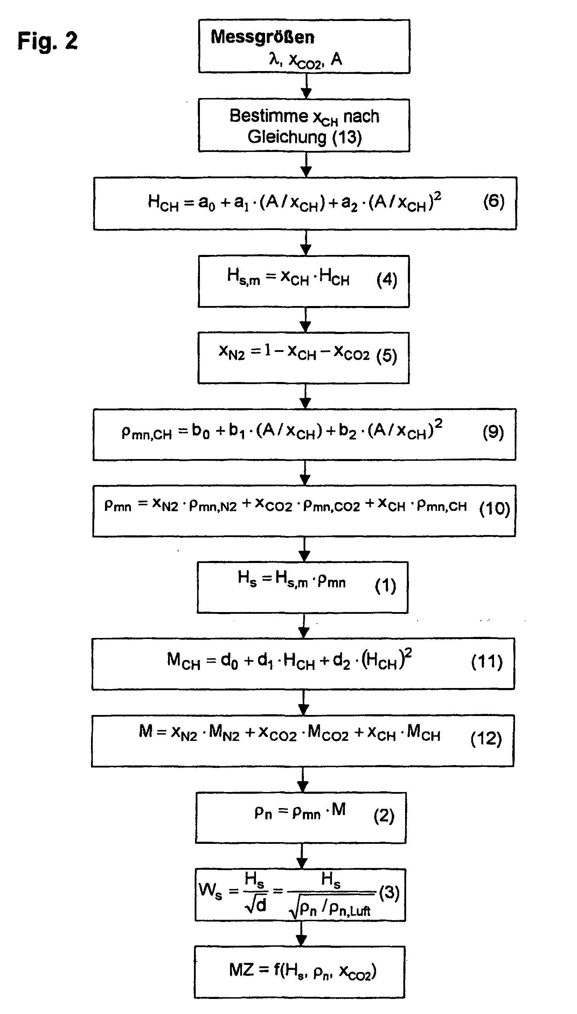

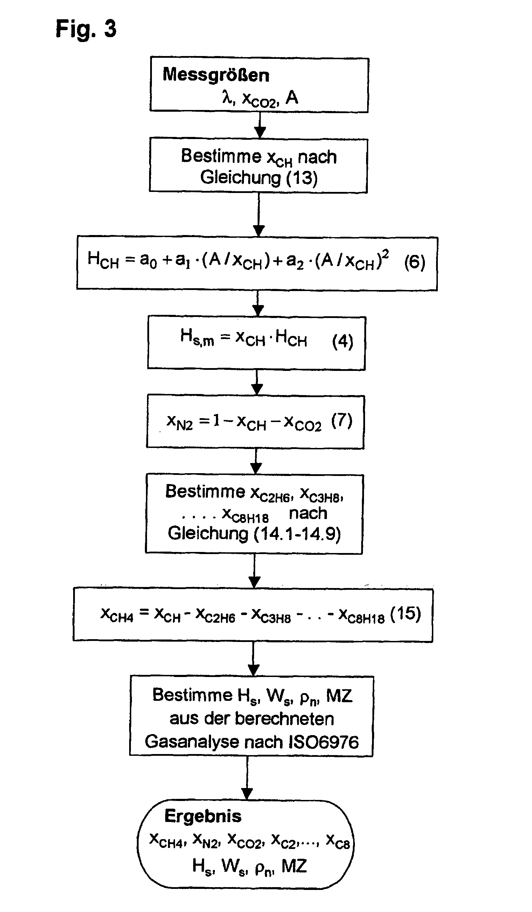

Die Erfindung ist ferner dadurch gekennzeichnet, dass die Gasbeschaffenheit im Schritt c) nach den Formel (13), (6), (4), (5), (9) (10), (1), (11), (12), (2) und (3) gemäß Fig. 2 oder nach den Formeln (13), (6), (4), (7), (14.1-14.9), (15) gemäß Fig. 3 berechnet wird.

Die bei dem ersten Ausführungbeispiel erläuterten Formeln (6), (9) und (7) beinhalten die Koeffizienten $a_0$, $a_1$, $a_2$, $b_0$, $b_1$, $b_2$ und $c_0$, $c_1$, $c_2$, die einmalig aus Messwerten aufgrund der Verfahrensschritte a) und b) an Referenzgasen bekannter Gaszusammensetzung bzw. die Gasbeschaffenheit bestimmt werden.

Üblicherweise werden hierzu drei oder mehr Referenzgase vermessen. Die Bestimmung der Koeffizienten erfolgt dann durch Anpassung an die Referenzgase indem die Fehlerquadratsumme durch lineare Regression minimiert wird. Diese Grundkalibration wird einmalig für ein Gerät durchgeführt. Für eine spätere Nachkalibrierung ist es ausreichend, eine Messung mit nur einem Referenzgas, z. B. reines Methan, durchzuführen (Ein-Punkt-Kalibrierung). Bei dieser Ein-Punkt-Kalibrierung werden dann nur die Koeffizienten $a_0$, $b_0$ und $c_0$ angepasst.

Die Erfindung beinhaltet ferner ein Verfahren zum Ermitteln der Energiemenge von Brenngas, insbesondere von Erdgas, dadurch gekennzeichnet, dass der Brennwert ermittelt, das Brenngas durch einen Volumenstromzähler geleitet und der Volumenstrom gemessen wird.

Die Erfindung befasst sich ferner mit einer Vorrichtung zum Ermitteln der Gasbeschaffenheit eines Brenngases, insbesondere von Erdgas, dadurch gekennzeichnet, dass Erdgas einer Sensoranordnung zugeführt wird, die im wesentlichen aus einer Strahlungsquelle für Infrarotstrahlung und mindestens zwei der Strahlungsquelle zugeordnete Strahlungsdetektoren sowie einem Sensor zum Erfassen der Wärmeleitfähigkeit besteht, und dass die Signale der Sensoranordnung einer Auswerteeinheit zugeführt werden, in der die Gasbeschaffenheit mittels einer Korrelation bestimmt wird.

Vorzugsweise ist jedem Strahlungsdetektor ein optischer Filter zugeordnet, der derart ausgewählt ist, dass zum einen der Anteil der Kohlenwasserstoffe, vorzugsweise bei einer Wellenlänge von 3,5 µm und zum anderen der Anteil des Kohlendioxids, vorzugsweise bei einer Wellenlänge von 4,3 µm sowie vorzugsweise der Anteil von Methan, vorzugsweise bei einer Wellenlänge von 7,9 µm erfasst wird.

Es ist besonders vorteilhaft, dass für die Messungen handelsübliche Sensoren eingesetzt werden können. Die Sensoren werden in Serienproduktion in großen Stückzahlen hergestellt und sind daher sehr preiswert und zuverlässig. Außerdem sind die Sensoren sehr kompakt, so dass sie problemlos in einem gemeinsamen Gehäuse, z. b. einem 19" Einschub, untergebracht werden können. Da die Sensoren direkt durchströmt werden und zusätzlich ein sehr kleines Volumen besitzen, ist die Ansprechzeit extrem gering, was vor allem bei der Regelung von Verbrennungsprozessen von großer Bedeutung ist.

Die Erfindung wird im folgenden anhand von zwei bevorzugten Ausführungsbeispielen mit Hilfe der beigefügten Zeichnungen näher erläutert. In dem ersten Ausführungsbeispiel werden die erforderlichen Gasbeschaffenheitskenngrößen aus den Messgrößen nach speziell zu diesem Zweck entwickelten Gleichungen berechnet. Im zweiten Ausführungsbeispiel wird in einem Zwischenschritt die Gasanalyse von insgesamt 12 Kompoenten berechnet. Aus dieser Gasanalyse werden dann die erforderlichen Gasbeschaffenheitskenngrößen nach bekannten Berechnungsverfahren abgeleitet.

In der Zeichnung zeigt in

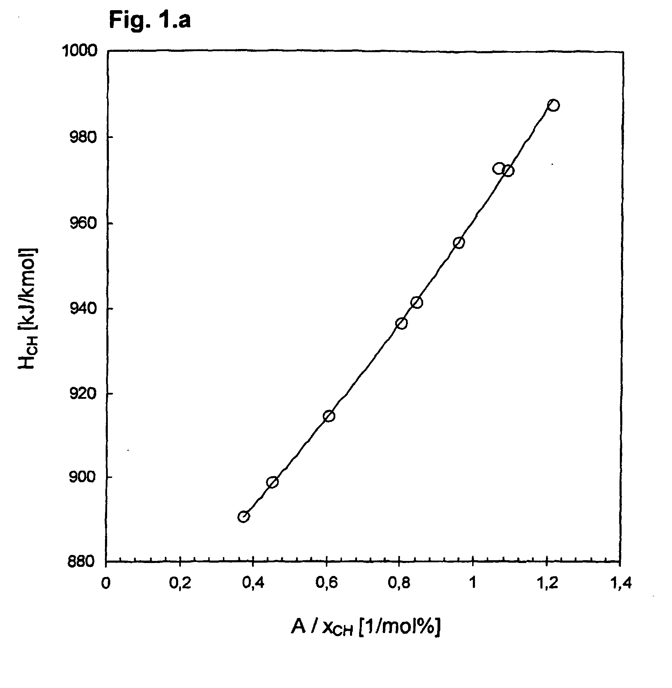

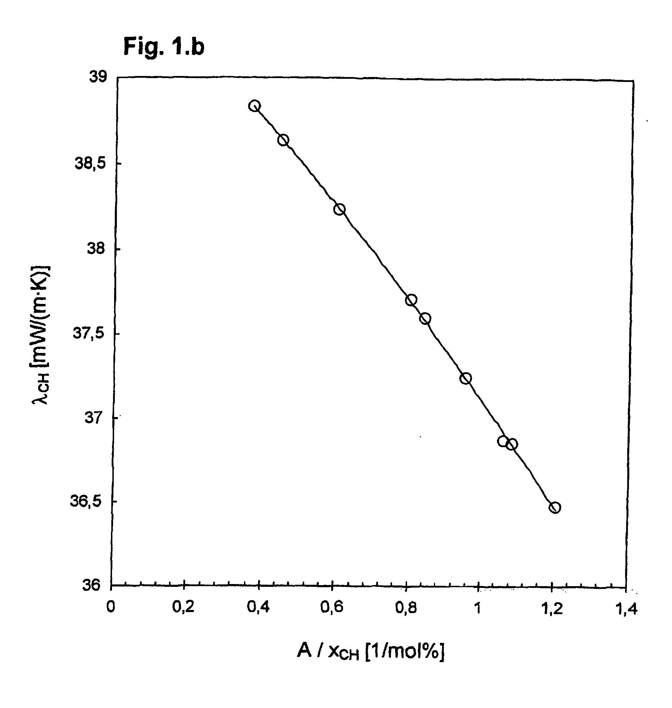

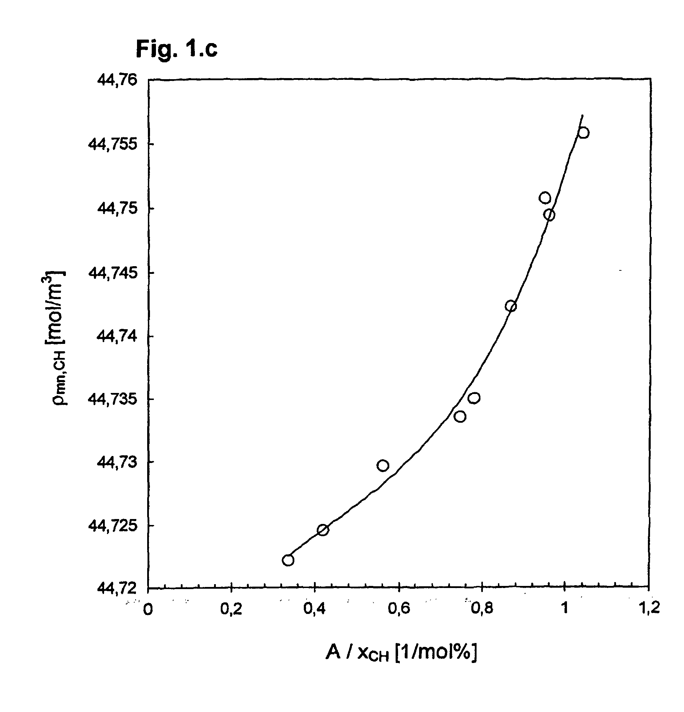

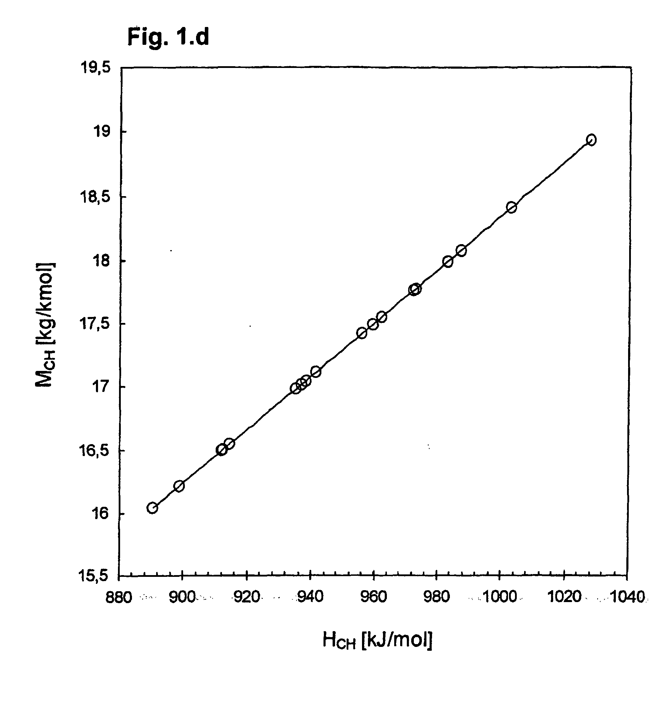

Fig. 1.a     den molaren Brennwert $H_{CH}$ des Kohlenwasserstoffgases als Funktion des Quotienten aus Infrarotabsorption A und Stoffmengenanteil $x_{CH}$ der Kohlenwasserstoffe für 8 natürliche Erdgase sowie Methan;

Fig. 1.b     die Wärmeleitfähigkeit $\lambda_{CH}$ des Kohlenwasserstoffgases als Funktion des Quotienten aus Infrarotabsorption A und des Stoffmengenanteils $x_{CH}$ der Kohlenwasserstoffe für 8 natürliche Erdgase sowie Methan;

Fig. 1.c     die molare Normdichte $\rho_{mn,CH}$ des Kohlenwasserstoffgases als Funktion des Quotienten aus Infrarotabsorption A und des Stoffmengenanteils $x_{CH}$ der Kohlenwasserstoffe für 8 natürliche Erdgase sowie Methan;

Fig. 1.d     die molare Masse $M_{CH}$ des Kohlenwasserstoffgases als Funktion des molaren Brennwertes $H_{CH}$ des Kohlenwasserstoffgases für 17 natürliche Erdgase sowie Methan;

Fig. 2     ein erstes Ausführungsbeispiel einer Berechnungsprozedur zur Bestimmung der Gasbeschaffenheitskenngrößen (Brennwert, Wobbezahl, Normdichte, Methanzahl) aus Wärmeleitfähigkeit $\lambda$, Stoffmengenanteil Kohlendioxid $x_{CO2}$, der sich direkt aus der Messung der Infrarotabsorption bei einer Wellenlänge von 4,3 µm ergibt und aus der Infrarotabsorption der Kohlenwasserstoffe A;

Fig. 3     ein zweites Ausführungsbeispiel einer Berechnungsprozedur zur Bestimmung der Gaszusammensetzung und der Gasbeschaffenheitskenngrößen (Brennwert, Wobbezahl, Normdichte, Methanzahl) aus Wärmeleitfähigkeit $\lambda$, Stoffmengenanteil Kohlendioxid $x_{CO2}$, der sich direkt aus der Messung der Infrarotabsorption bei einer Wellenlänge von etwa 4,3 µm ergibt und aus der Infrarotabsorption der Kohlenwasserstoffe A;

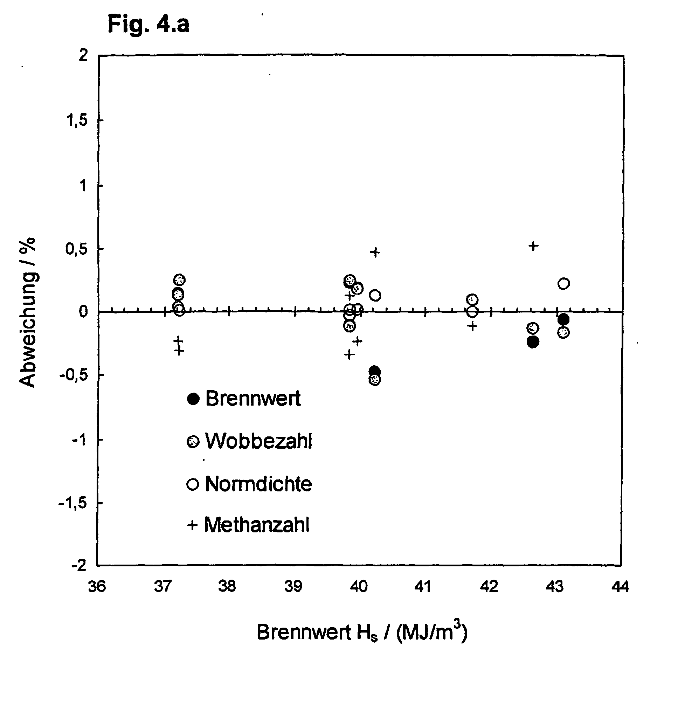

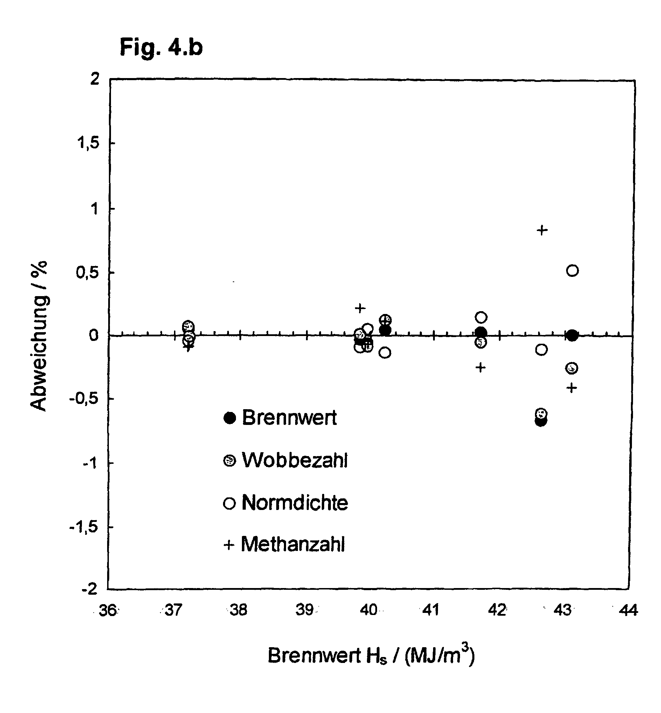

Fig. 4.a einen Vergleich von Gasbeschaffenheitskenngrößen (Brennwert, Wobbezahl, Normdichte, Methanzahl), die nach dem erfindungsgemäßen Verfahren (erstes Ausführungsbeispiel) bestimmt wurden, mit Werten, die aus der gaschromatographischen Analyse abgeleitet worden sind;

Fig. 4.b einen Vergleich von Gasbeschaffenheitskenngrößen (Brennwert, Wobbezahl, Normdichte, Methanzahl), die nach dem erfindungsgemäßen Verfahren (zweites Ausführungsbeispiel) bestimmt wurden, mit Werten, die aus der gaschromatographischen Analyse abgeleitet worden sind;

Fig. 5 die Ergebnisse eines Feldversuches. Aufgetragen ist der Brennwert der zum einem mit der erfindungsgemäßen Vorrichtung und zum anderen mit dem Kalorimeter gemessen wird über einem Zeitraum von einem Monat,

Fig. 6 eine schematische Ansicht der Messanordnung zur Bestimmung der Gasbeschaffenheit von Erdgasen;

Fig. 7 ein Diagramm mit der Darstellung des Brennwertes sowie den Signalen für ein beispielweisen Drei-Komponenten-Brennstoff, bestehend aus Methan ($CH_4$), Ethan ($C_2H_6$) und Stickstoff ($N_2$).

Erstes Ausführungsbeispiel:

Im folgenden wird als erstes Ausführungsbeispiel die Berechnungsprozedur, bzw das Korrelationsverfahren nach Fig. 2 beschrieben.

Als Eingangsgrößen werden die Wärmeleitfähigkeit $\lambda$, der Stoffmengenanteil Kohlendioxid $x_{CO2}$, der sich direkt aus der Messung der Infrarotabsorption bei einer Wellenlänge von etwa 4,3 $\mu$m ergibt und die Infrarotabsorption der Kohlenwasserstoffe A gemessen.

Der volumenbezogen Brennwert $H_s$ ergibt sich aus dem Produkt von molarem Brennwert $H_{s,m}$ und molarer Normdichte $\rho_{mn}$.

$$H_s = H_{s,m} \cdot \rho_{mn} \tag{1}$$

Die massenbezogene Normdichte $\rho_n$ ergibt sich aus dem Produkt von molarer Normdichte $\rho_{mn}$ und molarer Masse M.

$$\rho_n = \rho_{mn} \cdot M \tag{2}$$

Die Wobbezahl ergibt sich aus dem Quotient von Brennwert $H_s$ und der Wurzel aus der relativen Dichte. Die relative Dichte ist der Quotient aus der Normdichte des betreffenden Gases und der Normdichte von Luft.

$$W_s = \frac{H_s}{\sqrt{d}} = \frac{H_s}{\sqrt{\rho_n/\rho_{n,Luft}}} \qquad (\rho_{n,Luft} = 1.292923 \text{ kg/m}^3) \tag{3}$$

Erdgas besteht im wesentlichen aus Stickstoff, Kohlendioxid sowie einem Kohlenwasserstoffgas, im folgenden mit CH gekennzeichnet, das sich vorwiegend aus den Alkanen Methan bis Oktan zusammensetzt. Da der Stickstoffanteil und der Kohlendioxidanteil keinen Beitrag zum Brennwert liefern, ergibt sich der molare Brennwert $H_{s,m}$ des Erdgases aus dem Stoffmengenanteil $X_{CH}$ und dem Brennwert $H_{CH}$ ($H_{CH} = \Sigma x_{CHi} \cdot H_{CHi}$) des Kohlenwasserstoffgases zu:

$$H_{s,m} = x_{CH} \cdot H_{CH} \tag{4}$$

Die Summe der Stoffmengenanteile des Stickstoff, des Kohlendioxid und des Kohlenwasserstoffgases ergibt eins ($x_{N2} + x_{CO2} + x_{CH} = 1$).

Der Stoffengenanteil des Stickstoffs ergibt sich demnach zu:

$$x_{N2} = 1 - x_{CH} - x_{CO2} \tag{5}$$

Wie in Fig. 1.a dargestellt, lässt sich der molare Brennwert des Kohlenwasserstoffgases $H_{CH}$ als Funktion des Quotienten aus Infrarotabsorption der Kohlenwasserstoffe A und dem Stoffmengenanteil der Kohlenwasserstoffe $x_{CH}$ darstellen.

$$H_{CH} = a_0 + a_1 \cdot (A/x_{CH}) + a_2 \cdot (A/x_{CH})^2 \tag{6}$$

Dieser Sachverhalt lässt sich dadurch erklären, dass die Stoffmengenanteile der Alkane im Erdgas einer regelmäßigen Verteilung unterliegen. Die Infrarotabsorption A in Gleichung (6) wird mit dem Infrarotsensor bei der Wellenlänge von 3,5 µm gemessen.

In ähnlicher Weise lässt sich auch die Wärmeleitfähigkeit $\lambda_{CH}$ des Kohlenwasserstoffgases als Funktion des Quotienten $(A/x_{CH})$ darstellen. Dieser Zusammenhang ist in Fig. 1.b dargestellt.

$$\lambda_{CH} = C_0 + C_1 \cdot (A/x_{CH}) + C_2 \cdot (A/x_{CH})^2 \tag{7}$$

Die Wärmeleitfähigkeit $\lambda$ des Erdgases lässt sich in Abhängigkeit der Stoffmengenanteile $x_{N2}$, $x_{CO2}$ und $x_{CH}$ wie folgt darstellen.

$$\lambda = x_{N2} \cdot \lambda_{N2} + x_{CO2} \cdot \lambda_{CO2} + x_{CH} \tag{8}$$

Werte für die Wärmeleitfähigkeiten der reinen Stoffe $\lambda_{N2}$ und $\lambda_{CO2}$ können aus der Literatur entnommen werden.

In ähnlicher Weise lässt sich auch die molare Normdichte $\rho_{mn,CH}$ des Kohlenwasserstoffgases als Funktion des Quotienten $(A/x_{CH})$ darstellen. Dieser Zusammenhang ist in Fig. 1.c dargestellt.

$$\rho_{mn,CH} = b_0 + b_1 \cdot (A/x_{CH}) + b_2 \cdot (A/x_{CH})^2 \tag{9}$$

Die molare Normdichte $\rho_{mn}$ des Erdgases lässt sich in Abhängigkeit der Stoffmengenanteile $x_{N2}$, $x_{CO2}$ und $x_{CH}$ wie folgt darstellen.

$$\rho_{mn} = x_{N2} \cdot \rho_{mn,N2} + x_{CO2} \cdot \rho_{mn,CO2} + x_{CH} \cdot \rho_{mn,CH} \tag{10}$$

Werte für die molaren Normdichten der reinen Stoffe $\rho_{mn,N2}$ und $\rho_{mn,CO2}$ können aus der Literatur entnommen werden.

Die Molare Masse $M_{CH}$ des Kohlenwasserstoffgases läßt sich als Funktion des molaren Brennwertes $H_{CH}$ des Kohlenwasserstoffgases darstellen. Dieser Zusammenhang ist in Fig. 1.d dargestellt.

$$M_{CH} = d_0 + d_1 \cdot H_{CH} + d_2 \cdot (H_{CH})^2 \tag{11}$$

Die molare Masse M des Erdgases lässt sich in Abhängigkeit der Stoffmengenanteile $x_{N2}$, $x_{CO2}$ und $x_{CH}$ wie folgt darstellen.

$$M = x_{N2} \cdot M_{N2} + x_{CO2} \cdot M_{CO2} + x_{CH} \cdot M_{CH} \tag{12}$$

Werte für die molaren Massen der reinen Stoffe $M_{N2}$ und $M_{CO2}$ können aus der Literatur entnommen werden.

Durch Einsetzen von Gleichung (5) und Gleichung (7) in Gleichung (8) und Auflösen nach $x_{CH}$ lässt sich der Stoffmengenanteil des Kohlenwasserstoffgases $x_{CH}$ direkt aus den Messgrößen $\lambda$, $x_{CO2}$ und A ableiten.

$$x_{CH} = \sqrt{\frac{1}{4}\left[\frac{c_1 \cdot A - \lambda + x_{CO2}(\lambda_{CO2} - \lambda_{N2}) + \lambda_{N2}}{c_0 - \lambda_{N2}}\right]^2 - \frac{c_2 \cdot A^2}{(c_0 - \lambda_{N2})}} - \frac{1}{2} \cdot \frac{c_1 \cdot A - \lambda + x_{CO2}(\lambda_{CO2} - \lambda_{N2}) + \lambda_{N2}}{c_0 - \lambda_{N2}}$$

(13)

Aus dem Stoffmengenanteil des Kohlenwasserstoffgases kann dann unter Verwendung von Gleichung (6) der Brennwert $H_{CH}$ des Kohlenwasserstoffgases und anschließend der molare Brennwert des Erdgases aus Gleichung (4) berechnet werden.

Der Stoffmengenanteil des Stickstoffs $x_{N2}$ kann dann aus den Stoffmengenanteilen $x_{CH}$ und $x_{CO2}$ nach Gleichung (5) bestimmt werden.

Durch Anwenden der Gleichungen (9) und (10) kann die molare Normdichte $\rho_{mn}$ des Erdgases und durch Einsetzten in Gleichung (1) der volumenbezogen Brennwert $H_s$ des Erdgases bestimmt werden.

Durch Anwenden der Gleichungen (11) und (12) kann die molare Masse M des Erdgses und durch Einsetzen in Gleichung (2) die massenbezogene Normdichte des Erdgase bestimmt werden.

Die Wobbezahl ergibt sich aus Gleichung (3).

Aus der Literatur (P. Schley und H. Frieling: Bestimmung der Methanzahl aus Gasbeschaffenheitskenngrößen. gwf-Gas/Erdgas 141 (2000) Nr. 1, S. 28-33) ist ferner ein Berechnungsverfahren bekannt, mit dem sich die Methanzahl aus dem Brennwert $H_s$, der Normdichte $\rho_n$ und dem Stoffmengenanteil $x_{CO2}$ bestimmen lässt. Eine schematische Darstellung der Berechnungsprozedur ist in Fig. 2 angegeben.

Die Koeffizienten $d_0$, $d_1$, $d_2$ in Gleichungen (11) werden einmalig anhand der Analyse mehrerer Referenzgase mit bekannter Gaszusammensetzung bzw. Gasbeschaffenheit bestimmt. Die Bestimmung der Koeffizienten erfolgt durch Anpassung an die Referenzgase indem die Fehlerquadratsumme durch lineare Regression minimiert wird.

Das beschriebene Berechnungsverfahren beinhaltet ferner die Koeffizienten $a_0$, $a_1$, $a_2$, $b_0$, $b_1$, $b_2$ und $c_0$ $c_1$, $c_2$ in den Gleichungen (6), (9) und (7) die durch eine einmalige Grundkalibration bestimmt werden. Die Kalibration erfolgt durch Messungen (Verfahrensschritte a) und b)) an Referenzgasen, deren Gaszusammensetzung bzw. Gasbeschaffenheit bekannt ist. Üblicherweise werden hierzu drei oder mehr Referenzgase vermessen. Die Bestimmung der Koeffizienten erfolgt dann durch Anpassung an die Referenzgase indem die Fehlerquadratsumme durch lineare Regression minimiert wird. Diese Grundkalibration wird einmalig für ein Gerät durchgeführt. Für eine spätere Nachkalibrierung ist es ausreichend, eine Messung mit nur einem Referenzgas, z. B. reines Methan, durchzuführen (Ein-Punkt-Kalibrierung). Bei dieser Ein-Punkt-Kalibrierung werden dann nur die Koeffizienten $a_0$, $b_0$, und $c_0$ angepasst.

Das Verfahren wurde im Labor an insgesamt 8 verschiedenen Erdgasen getestet. In Fig. 4.a sind die prozentualen Abweichungen der nach der ersten Ausführungsform des erfindungsgemäßen Verfahrens ermittelten Gasbeschaffenheitskenngrößen (Brennwert, Wobbezahl, Normdichte und Methanzahl) und den aus der gaschromatographischen Analyse abgeleiteten Werten dargestellt. In der Regel sind die Abweichungen für den Brennwert kleiner als ± 0,25 %. Nur bei einer Gasprobe beträgt die Abweichung 0,5 %.

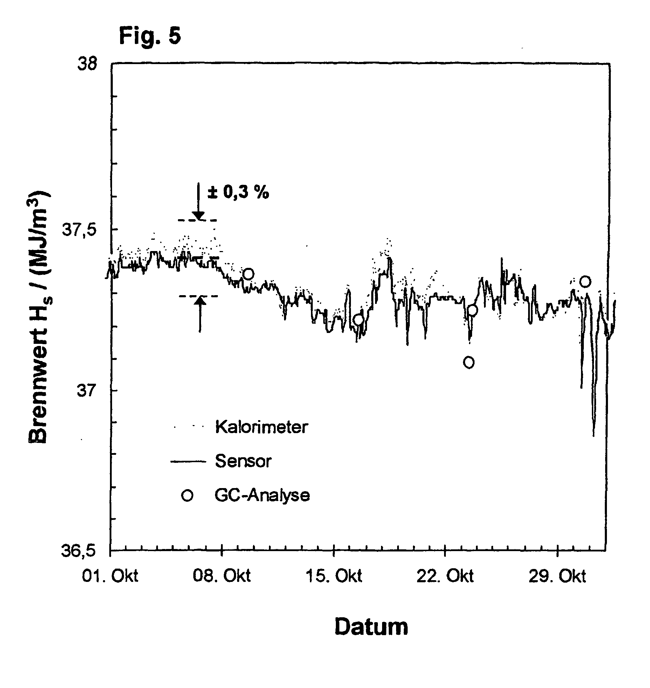

Die Langzeitstabilität wurde anhand eines Feldversuches untersucht. Dabei wurden die Messsignale kontinuierlich aufgenommen und mit einem Kalorimeter, das zu Abrechnungszwecken eingesetzt wird, verglichen. Das Ergebnis ist in Fig. 5 beispielhaft für einen Monat dargestellt. Das Bild zeigt, das die Übereinstimmung des aus der Sensoranordnung abgeleiteten Brennwertes mit dem gemessenen Brennwert des Kalorimeters besser als 0,2 % ist. Damit stimmen beide Verfahren innerhalb der Messunsicherheit des Kalorimeters (0,3 %) überein. Während des gesamten Feldversuches, der über einen Zeitraum von sechs Monaten durchgeführt wurde, konnte keine signifikante Drift des Messsignals beobachtet werden.

Zweites Ausführungsbeispiel:

Im folgenden wird die Berechnungsprozedur, bzw. das Korrelationsverfahren nach Fig. 3 beschrieben.

Als Eingangsgrößen werden die Wärmeleitfähigkeit $\lambda$, der Stoffmengenanteil Kohlendioxid $x_{CO2}$, der sich direkt aus der Messung der Infrarotabsorption bei einer Wellenlänge von etwa 4,3 µm ergibt und die Infrarotabsorption der Kohlenwasserstoffe A gemessen.

Die ersten Berechnungsschritte erfolgen zunächst analog zu dem ersten Ausführungsbeispiel.

Der Stoffmengenanteil des Kohlenwasserstoffgases $x_{CH}$ ergibt sich aus Gleichung (13). Durch Ausführen der Gleichungen (6) und (4) wird der molare Brennwert des Erdgases berechnet.

Der Stoffmengenanteil des Stickstoffs $x_{N2}$ kann dann aus den Stoffmengenanteilen $x_{CH}$ und $x_{CO2}$ nach Gleichung (5) bestimmt werden.

Aus dem Brennwert $H_{CH}$ und den Stoffmengenanteilen $x_{CH}$ des Kohlenwasserstoffgases lassen sich im folgenden die Stoffmengenanteile der einzelnen Alkane von Ethan bis Oktan ableiten.

$$x_{C2H6} = \left\{ \alpha_1 (H_{CH} - H_{CH4}) + \beta_1 (H_{CH} - H_{CH4})^2 \right\} \qquad (14.1)$$

$$x_{C3H8} = \left\{ \alpha_2 (H_{CH} - H_{CH4}) + \beta_2 (H_{CH} - H_{CH4})^2 \right\} \qquad (14.2)$$

$$x_{n-C4H10} = \left\{ \alpha_3 (H_{CH} - H_{CH4}) + \beta_3 (H_{CH} - H_{CH4})^2 \right\} \qquad (14.3)$$

$$x_{i-C4H10} = \left\{ \alpha_4 (H_{CH} - H_{CH4}) + \beta_4 (H_{CH} - H_{CH4})^2 \right\} \qquad (14.4)$$

$$x_{n-C5H12} = \left\{ \alpha_5 (H_{CH} - H_{CH4}) + \beta_5 (H_{CH} - H_{CH4})^2 \right\} \qquad (14.5)$$

$$x_{i-C5H12} = \left\{ \alpha_6 (H_{CH} - H_{CH4}) + \beta_6 (H_{CH} - H_{CH4})^2 \right\} \qquad (14.6)$$

$$x_{n-C6H14} = \left\{ \alpha_7 (H_{CH} - H_{CH4}) + \beta_7 (H_{CH} - H_{CH4})^2 \right\} \qquad (14.7)$$

$$x_{n-C7H16} = \left\{ \alpha_8 (H_{CH} - H_{CH4}) + \beta_8 (H_{CH} - H_{CH4})^2 \right\} \qquad (14.8)$$

$$x_{n-C8H18} = \left\{ \alpha_9 (H_{CH} - H_{CH4}) + \beta_9 (H_{CH} - H_{CH4})^2 \right\} \qquad (14.9)$$

Die Koeffizienten $\alpha_1$ bis $\beta_9$ werden einmalig anhand der Analyse mehrerer Referenzgase mit bekannter Gaszusammensetzung bzw. Gasbeschaffenheit bestimmt. Die Bestimmung der Koeffizienten erfolgt durch Anpassung an die Referenzgase indem die Fehlerquadratsumme durch lineare Regression minimiert wird.

Der Stoffmengenanteil des Methans ergibt sich dann zu:

$$x_{CH4} = x_{CH} - x_{C2H6} - x_{C3H8} - x_{n-C4H10} - x_{i-C4H10} - x_{n-C5H12} - x_{n-C6H14} - x_{n-C7H16} - x_{n-C8H18} \qquad (15)$$

Die so bestimmte Gasanalyse von insgesamt 12 Komponenten ($N_2$, $CO_2$, 10 Alkane) kann nun verwendet werden um weitere Gasbeschaffenheitskenngrößen, wie z. B. den volumetrischen Brennwert $H_s$, die Wobbezahl $W_s$, die Normdichte $\rho_n$ oder die Methanzahl MZ abzuleiten. Die Berechnung der Größen $H_s$, $W_s$ und $\rho_n$ erfolgt nach dem internationalen Standard ISO6976. Eine schematische Darstellung der Berechnungsprozedur ist in Fig. 3 angegeben.

Das Verfahren wurde im Labor an insgesamt 8 verschiedenen Erdgasen getestet. In Fig. 4.b sind die prozentualen Abweichungen der mit der zweiten Ausführungsform des erfindungsgemäßen Verfahrens ermittelten Gasbeschaffenheitskenngrößen (Brennwert, Wobbezahl, Normdichte und Methanzahl) und den aus der gaschromatographischen Analyse abgeleiteten Werten dargestellt. In der Regel sind die Abweichungen für den Brennwert kleiner als ± 0,1 %. Nur bei einer Gasprobe beträgt die Abweichung 0,6 %.

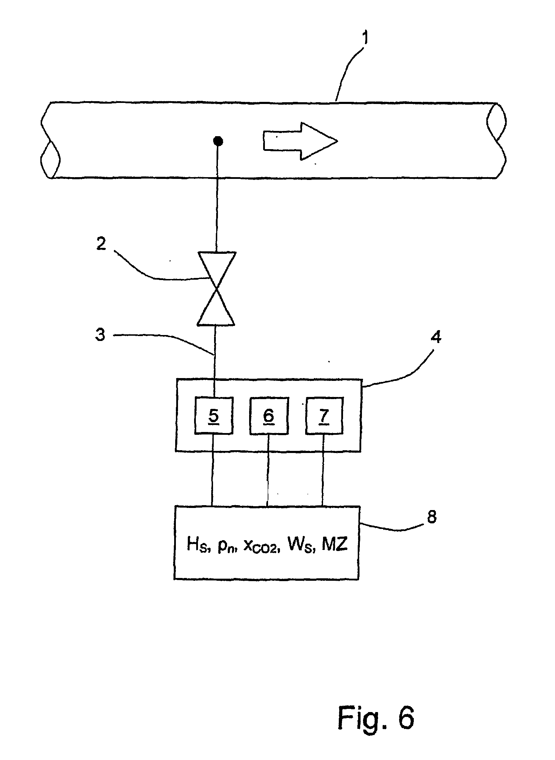

Eine schematische Darstellung der erfindungsgemäßen Vorrichtung ist in Fig. 6 angegeben. Ein Teilstrom des Erdgases wird kontinuierlich aus einer Haupttransportleitung 1 entnommen und über einen Druckminderer 2 in einer Zweigleitung 3 auf etwa 20-100 mbar, d. h. im wesentlichen auf Atmosphärendruck entspannt und einer Sensoranordnung 4 zugeführt. Die Sensoranordnung 4 besteht im wesentlichen aus einer nicht dargestellten Strahlungsquelle für Infrarotstrahlung und zwei der Strahlungsquelle zugeordneten Strahlungsdetektoren 5, 6. Ein dritter Sensor 7 misst die Wärmeleitfähigkeit. Die Sensoren 5, 6, 7 nehmen kontinuierlich die Messsignale auf, die von einer Auswerteeinheit 8 in Form einer Elektronik (herkömmliche Leiterkarte) direkt ausgewertet werden.

Die Temperatur wird auf nicht dargestellte Weise gemessen, so dass die Möglichkeit besteht, die Messwerte auf Referenzbedingen umzurechnen. Falls die Temperatur in der Messumgebung stark schwankt, ist es vorteilhaft, die Temperatur auf einen Wert, z. B. 50°C einzustellen bzw. zu regeln.

In Fig. 7 ist grafisch dargestellt, wie der Brennwert eines 3-Komponenten-Gemisches aus zwei Eingangsgrößen, und zwar der Infrarotabsorption A und der Wärmeleitfähigkeit $\lambda$ bestimmt werden kann und welche resultierende Gesamtunsicherheit sich für den Brennwert ergibt.

Die Abkürzungen in Fig. 7 werden im folgenden erläutert:

$H_s$ Sollwert des Brennwertes
A Sollwert des IR-Signals
$\lambda$ Sollwert der Wärmeleitfähigkeit

In Fig. 7 ist auf der Abzissenachse der Stoffmengenanteil des Stickstoffs ($x_{N2}$) und auf der Ordinatenachse der Stoffmengenanteil des Ethans ($x_{C2H6}$) aufgetragen. Die dritte nicht dargestellte Komponente ist Methan ($x_{CH4}$). Der Stoffmengenanteil des Methans entspricht dem Rest des Anteiles des Gemisches um zu 100 mol% zu gelangen.

In dem Diagramm wurde als Bezugspunkt willkürlich die Zusammensetzung $x_{N2}$ = 4 mol % und $x_{C2H6}$ = 6 mol % gewählt. Durch diesen Bezugspunkt wird eine Linie konstanten Brennwertes eingezeichnet ($H_s$ = const.). Parallel dazu sind Linien für die Brennwerte $H_s$ +0,2 %, Hs +0,4 %, ... $H_s$ +1% bzw. $H_s$ -0,2 %, $H_s$ -0,4 %, ... $H_s$ -1% eingezeichnet. Ebenfalls durch den Bezugspunkt verlaufen Linien, für die die Eingangsgrößen A und $\lambda$ konstante Werte annehmen. Die äußeren, dünneren Linien, geben dabei das Unsicherheitsband der Eingangsgrößen wieder. Die resultierende Unsicherheit für den Brennwert ergibt sich aus der Schnittfläche der Unsicherheitsbänder der Eingangsgrößen (schattiertes Parallelogramm in Fig. 7). Die Unsicherheit für den Brennwert beträgt etwa 0,4 %.

Die drei Komponenten des Beispiel-Gemisches sind die wichtigsten Bestandteile des Erdgases. Erdgas weist eine Vielzahl von weiteren Komponenten auf. Allerdings lassen sich die Messergebnisse eines Gemisches von mehr als drei Komponenten nicht mehr grafisch darstellen.

Der Kohlendioxidanteil im Erdgas ist gering und unterliegt nur geringen Schwankungen, so dass hier ein Mittelwert vorgegeben werden kann. Der Anteil der n-Alkane im Erdgas nimmt mit zunehmender Anzahl an C-Atomen ab, so dass diese nur bis zum Hexan oder Oktan berücksichtigt werden brauchen. Zudem unterliegen die Anteile der n-Alkane einer regelmäßigen Verteilung und können über eine geeignete Korrelation berechnet werden (z.B. aus dem Infrarotsignal). Aufgrund dieser Überlegungen lassen sich die in Fig. 7 dargestellten resultierenden Unsicherheiten für den Brennwert näherungsweise auch auf natürliche Erdgase übertragen.

**Patentansprüche**

**1.** Verfahren zum Ermitteln der Gasbeschaffenheit von Brenngas, insbesondere von Erdgas, wobei

a) zumindest ein Teil des Brenngases einer Infrarotstrahlung ausgesetzt wird und für zwei unterschiedliche Wellenlängen oder spektrale Bereiche der jeweils vom Brenngas absorbierte Anteil der Infrarotstrahlung gemessen wird,
b) die Wärmeleitfähigkeit gemessen wird und
c) aus den drei Messwerten die Gasbeschaffenheit rechnerisch bestimmt wird.

**2.** Verfahren nach Anspruch 1,
**dadurch gekennzeichnet, dass** die Wellenlängen oder spektralen Bereiche derart ausgewählt werden, dass zum einen der Anteil der Kohlenwasserstoffe, vorzugsweise bei einer Wellenlänge von 3,5 $\mu$m und zum anderen der Anteil des Kohlendioxids, vorzugsweise bei einer Wellenlänge von 4,3 $\mu$m erfasst wird.

**3.** Verfahren nach Anspruch 2,

**dadurch gekennzeichnet, dass** zusätzlich der vom Brenngas absorbierte Anteil der Infrarotstrahlung für eine weitere Wellenlänge oder spektralen Bereich gemessen wird, wobei die Wellenlänge oder der spektrale Bereich so gewählt wird, dass der Anteil von Methan, vorzugsweise bei einer Wellenlänge von 7,9 μm, erfasst wird.

4. Verfahren nach einem der Ansprüche 1 bis 3,
**dadurch gekennzeichnet, dass** der Anteil der Infrarotstrahlung sowie die Wärmeleitfähigkeit unter Referenzbedingungen in einer gemeinsamen Messumgebung gemessen werden.

5. Verfahren nach einem der Ansprüche 1 bis 4,
**dadurch gekennzeichnet, dass** im Schritt a) oder b) die Temperatur und der Druck gemessen oder konstant gehalten werden.

6. Verfahren nach einem der Ansprüche 1 bis 5,
**dadurch gekennzeichnet, dass** die Gasbeschaffenheit im Schritt c) nach den Formeln (13), (6), (4), (5), (9) (10), (1), (11), (12), (2) und (3) gemäß Fig. 2 oder nach den Formeln (13), (6), (4), (7), (14.1-14.9), (15) gemäß Fig. 3 berechnet wird.

7. Verfahren nach Anspruch 6,
**dadurch gekennzeichnet, dass** die Koeffizienten $a_0$, $a_1$, $a_2$, $b_0$, $b_1$, $b_2$ und $c_0$, $c_1$, $c_2$ für die Gleichungen (6), (9) und (7) aus Messwerten aufgrund der Schritte a) und b) mit mehreren Referenzgasen bekannter Gasbeschaffenheit bestimmt werden.

8. Verfahren zum Ermitteln der Energiemenge von Brenngas, insbesondere von Erdgas nach einem der Ansprüche 1 bis 7,
**dadurch gekennzeichnet, dass** der Brennwert ermittelt wird und das Brenngas durch einen Volumenstromzähler geleitet und der Volumenstrom gemessen wird.

9. Vorrichtung zum Ermitteln der Gasbeschaffenheit eines Brenngases, insbesondere von Erdgas,
**dadurch gekennzeichnet, dass** das Brenngas einer Sensoranordnung (4) zugeführt wird, die im wesentlichen aus einer Strahlungsquelle für Infrarotstrahlung und mindestens zwei der Strahlungsquelle zugeordnete Strahlungsdetektoren (5, 6) sowie einem Sensor (7) zum Erfassen der Wärmeleitfähigkeit besteht, und dass die Signale der Sensoranordnung einer Auswerteeinheit zugeführt werden, in der die Gasbeschaffenheit mittels einer Korrelation bestimmt wird.

10. Vorrichtung nach Anspruch 9,
**dadurch gekennzeichnet, dass** jedem Strahlungsdetektor (5,6) ein optischer Filter zugeordnet ist, der derart ausgewählt ist, dass zum einen der Anteil der Kohlenwasserstoffe, vorzugsweise bei einer Wellenlänge von 3,5 μm und zum anderen der Anteil des Kohlendioxids, vorzugsweise bei einer Wellenlänge von 4,3 μm sowie vorzugsweise der Anteil von Methan, vorzugsweise bei einer Wellenlänge von 7,9 μm erfasst wird.

**Claims**

1. Method for determining the gaseous quality of a fuel gas, in particular natural gas, wherein

a) at least part of the fuel gas is exposed to infrared radiation and the proportion of the infrared radiation absorbed by the fuel gas is measured for two different wave lengths or spectral ranges,
b) the thermal conductivity is measured and
c) the gas quality is calculated from the three measurements.

2. Method according to claim 1,
**characterised in that** the wave lengths or spectral ranges are chosen so that the proportion of hydrocarbons is measured, preferably at a wave length of 3.5 μm, and the proportion of carbon dioxide is measured, preferably at a wave length of 4.3 μm.

3. Method according to claim 2,
**characterised in that** the proportion of infrared radiation absorbed by the fuel gas is also measured for a further wave length or spectral range, the wave length or spectral range being selected so that the proportion of methane is measured, preferably at a wave length of 7.9 μm.

4. Method according to any one of claims 1 through 3,
**characterised in that** the proportion of infrared radiation and the thermal conductivity are measured under reference conditions in a common measuring environment.

5. Method according to any one of claims 1 through 4,
**characterised in that** the temperature and the pressure are measured or kept constant in step a) or b).

6. Method according to any one of claims 1 through 5,
**characterised in that** the gas quality is calculated in step c) according to the formulae (13), (6), (4), (5), (9), (10), (1), (11), (12), (2) and (3) in accordance with Fig. 2 or according to the formulae (13), (6), (4), (7), (14.1-14.9), (15) according to Fig. 3.

7. Method according to claim 6,
**characterised in that** the coefficients $a_0$, $a_1$, $a_2$, $b_0$, $b_1$, $b_2$ and $c_0$, $c_1$, $c_2$ are determined for the equations (6), (9) and (7) from the values measured in steps a) and b) with several reference gases of known gas quality.

8. Method for determining the energy quantity of fuel gas, in particular of natural gas according to any one of claims 1 through 7,
**characterised in that** the gross calorific value is determined and that the fuel gas is passed through a volumetric meter and the volumetric gas flow is measured.

9. Device for determining the gas quality of a fuel gas, in particular natural gas,
**characterised in that** the fuel gas is fed to a sensor arrangement (4) which mainly consists of a source of radiation for infrared radiation and at least two radiation detectors (5, 6) assigned to the source of radiation as well as a sensor (7) for measuring the thermal conductivity, and that the signals from the sensor arrangement are transmitted to an evaluation unit in which the gas quality is determined by correlation.

10. Device according to claim 9,
**characterised in that** an optical filter is assigned to each radiation detector (5, 6) which is chosen so that the proportion of hydrocarbons is measured, preferably at a wave length of 3.5 μm, and the proportion of carbon dioxide is measured, preferably at a wave length of 4.3 μm, and preferably the proportion of methane is measured, preferably at a wave length of 7.9 μm.

**Revendications**

1. Procédé pour déterminer la nature de gaz combustibles, en particulier de gaz naturel, où

a) au moins une partie du gaz combustible est soumise à un rayonnement infrarouge et, pour deux longueurs d'ondes différentes ou régions du spectre, la partie du rayonnement infrarouge absorbée par le gaz combustible à chaque fois est mesurée,
b) la conductivité thermique est mesurée, et
c) la nature du gaz est déterminée par calcul à partir des trois valeurs de mesure.

2. Procédé suivant la revendication 1,
**caractérisé par le fait que** les longueurs d'ondes ou régions du spectre sont choisies de la sorte que, d'une part, la part des hydrocarbures est saisie, de préférence à une longueur d'onde de 3,5 μm, et, d'autre part, la part du dioxyde de carbone est saisie, de préférence à une longueur d'onde de 4,3 μm.

3. Procédé suivant la revendication 2,
**caractérisé par le fait que** la partie du rayonnement infrarouge absorbée par le gaz combustible et de plus mesurée pour une autre longueur d'ondes ou région du spectre, la longueur d'ondes ou la région du spectre étant choisie de la sorte que la part du méthane est saisie, de préférence à une longueur d'onde de 7,9 μm.

4. Procédé suivant l'une des revendications 1 à 3,
**caractérisé par le fait que** la part du rayonnement infrarouge ainsi que la conductivité thermique sont mesurées dans des conditions de référence dans un environnement commun de mesurage.

5. Procédé suivant l'une des revendications 1 à 4,
   **caractérisé par le fait que**, dans l'étape a) ou b), la température et la pression sont mesurées ou maintenues constantes.

6. Procédé suivant l'une des revendications 1 à 5,
   **caractérisé par le fait que** la nature du gaz, dans l'étape c), est calculée suivant les équation (13), (6), (4), (5), (9), (10), (1), (11), (12), (2) et (3) selon la figure 2 ou suivant les équations (13), (6), (4), (7), (14.1-14.9), (15) selon la figure 3.

7. Procédé suivant la revendication 6, **caractérisé par le fait que** les coefficients $a_0$, $a_1$, $a_2$, $b_0$, $b_1$, $b_2$ et $c_0$, $c_1$, $c_2$ pour les équations (6), (9) et (7) sont déterminés à partir des étapes a) et b) avec plusieurs gaz de référence dont la nature est connue.

8. Procédé pour déterminer la quantité d'énergie de gaz combustibles, en particulier de gaz naturel, suivant l'une des revendications 1 à 7,
   **caractérisé par le fait que** le pouvoir calorifique supérieur est déterminé, que le gaz combustible est fait passer à travers d'un compteur volumétrique et que le débit volumique est mesuré.

9. Dispositif pour déterminer la nature d'un gaz combustible, en particulier de gaz naturel,
   **caractérisé par le fait que** le gaz combustible est amené à une disposition de capteurs (4), consistant essentiellement d'une source de rayonnement pour le rayonnement infrarouge et d'au moins deux détecteurs de rayonnement (5, 6), attribués à la source de rayonnement, ainsi que d'un capteur (7) pour la saisie de la conductivité thermique, et que les signaux de la disposition de capteurs sont amenés à une unité d'exploitation de données où la nature du gaz est déterminée à l'aide d'une corrélation.

10. Dispositif suivant la revendication 9,
    **caractérisé par le fait qu'**à chaque détecteur de rayonnement (5, 6) est attribué un filtre optique qui est choisi de la sorte que, d'une part, la part des hydrocarbures est saisie, de préférence à une longueur d'onde de 3,5 µm, et, d'autre part, la part du dioxyde de carbone est saisie, de préférence à une longueur d'onde de 4,3 µm ainsi que, de préférence, la part du méthane est saisie, de préférence à une longueur d'onde de 7,9 µm.

**Fig. 1.a**

**Fig. 1.b**

**Fig. 1.c**

## Fig. 1.d

**Fig. 2**

$$\boxed{\begin{array}{c}\textbf{Messgrößen} \\ \lambda,\ x_{CO2},\ A\end{array}}$$

$$\boxed{\begin{array}{c}\text{Bestimme } x_{CH} \text{ nach} \\ \text{Gleichung (13)}\end{array}}$$

$$\boxed{H_{CH} = a_0 + a_1 \cdot (A/x_{CH}) + a_2 \cdot (A/x_{CH})^2 \qquad (6)}$$

$$\boxed{H_{s,m} = x_{CH} \cdot H_{CH} \qquad (4)}$$

$$\boxed{x_{N2} = 1 - x_{CH} - x_{CO2} \quad (5)}$$

$$\boxed{\rho_{mn,CH} = b_0 + b_1 \cdot (A/x_{CH}) + b_2 \cdot (A/x_{CH})^2 \qquad (9)}$$

$$\boxed{\rho_{mn} = x_{N2} \cdot \rho_{mn,N2} + x_{CO2} \cdot \rho_{mn,CO2} + x_{CH} \cdot \rho_{mn,CH} \qquad (10)}$$

$$\boxed{H_s = H_{s,m} \cdot \rho_{mn} \qquad (1)}$$

$$\boxed{M_{CH} = d_0 + d_1 \cdot H_{CH} + d_2 \cdot (H_{CH})^2 \qquad (11)}$$

$$\boxed{M = x_{N2} \cdot M_{N2} + x_{CO2} \cdot M_{CO2} + x_{CH} \cdot M_{CH} \qquad (12)}$$

$$\boxed{\rho_n = \rho_{mn} \cdot M \qquad (2)}$$

$$\boxed{W_s = \dfrac{H_s}{\sqrt{d}} = \dfrac{H_s}{\sqrt{\rho_n / \rho_{n,Luft}}} \quad (3)}$$

$$\boxed{MZ = f(H_s,\ \rho_n,\ x_{CO2})}$$

## Fig. 3

Messgrößen
$\lambda$, $x_{CO2}$, A

Bestimme $x_{CH}$ nach
Gleichung (13)

$$H_{CH} = a_0 + a_1 \cdot (A / x_{CH}) + a_2 \cdot (A / x_{CH})^2 \quad (6)$$

$$H_{s,m} = x_{CH} \cdot H_{CH} \quad (4)$$

$$x_{N2} = 1 - x_{CH} - x_{CO2} \quad (7)$$

Bestimme $x_{C2H6}$, $x_{C3H8}$,
. . . . $x_{C8H18}$ nach
Gleichung (14.1-14.9)

$$x_{CH4} = x_{CH} - x_{C2H6} - x_{C3H8} - . . - x_{C8H18} \quad (15)$$

Bestimme $H_s$, $W_s$, $\rho_n$, MZ
aus der berechneten
Gasanalyse nach ISO6976

Ergebnis
$x_{CH4}$, $x_{N2}$, $x_{CO2}$, $x_{C2}$,..., $x_{C8}$
$H_s$, $W_s$, $\rho_n$, MZ

# Fig. 4.a

# Fig. 4.b

Fig. 5

Fig. 6

## Fig. 7